# EUROPEAN PATENT APPLICATION

(11) **EP 4 825 325 A2**
(43) Date of publication of application: **21.10.2026**
(21) Application number: 26203235.2
(22) Date of filing: 30.11.2022
(51) Int. Cl.: A61B 10/02

(54) **MULTI-FACETED BIOPSY NEEDLE TIPS AND NEEDLES, NEEDLE SETS, AND DEVICES INCORPORATING THE SAME**

(30) Priority: 01.12.2021 US 202163284820 P
(62) Document number(s) of the earlier application(s) in accordance with Art. 76 EPC: 22843576.4 / 4 440 444
(71) Applicant: Hologic, Inc., Marlborough, MA 01752 (US)
(72) Inventor: ZHOU, Yi, Marlborough, 01752 (US); BLOZNALIS, Peter, Marlborough, 01752 (US)
(74) Representative: Carpmaels & Ransford LLP

(57) **Abstract**

A multi-faceted soft tissue piercing element includes three distal planar cutting edges converging to form a sharp, distal point of the tissue piercing element. Each distal cutting edge forms a first angle with respect to a central longitudinal axis of the tissue piercing element. The tissue piercing element also includes three proximal planar cutting edges. Each proximal cutting edge extends proximally from a respective one of the three distal planar cutting edges. Each proximal cutting edge forms a second angle relative to the central longitudinal axis of the tissue piercing element. The second angle is larger than the first angle.

## Description

### Cross-Reference to Related Applications

This application claims priority to U.S. Provisional Application No. 63/284,820, filed December 1, 2021, the entire contents of which is incorporated by reference herein.

### Technical Field

The present disclosure generally relates to the field of soft tissue sampling and harvesting. More specifically, the disclosure relates to needle tip designs for biopsy needles, needle sets, and/or devices capable of sampling soft tissue.

### Introduction

The section headings used herein are for organizational purposes only and are not to be construed as limiting the subject matter described in any way.

In the practice of diagnostic medicine, it is often necessary or desirable to perform a biopsy, or to sample selected tissue from a living patient for medical evaluation. Cytological and histological studies of the biopsy sample can then be performed as an aid to the diagnosis and treatment of disease. Biopsies can be useful in diagnosing and treating various forms of cancer, including, for example, breast cancer, as well as other diseases in which a localized area of affected tissue can be identified.

Preoperative diagnosis with a percutaneous biopsy is generally preferred over surgical biopsy. With a percutaneous biopsy, a surgeon takes a tissue sample through the skin with a needle set, as opposed to making an actual surgical excision. One known needle set includes an elongate outer cannula (needle) having a pointed tissue piercing (penetrating) tip and a receiving aperture defined near its distal end (adjacent to the tissue piercing tip), and an inner cannula having an open distal end surrounded by an annular cutting blade. The inner cannula is slidably disposed within the outer cannula so that it can close the tissue receiving aperture, thereby cutting tissue prolapsing into the lumen of the outer cannula (needle) through the tissue receiving aperture.

Percutaneous image-guided needle biopsy, for example, has become the preferred biopsy method in the management of suspicious breast lesions detected by screening or during the assessment of clinical abnormalities. Percutaneous image-guided needle biopsy is recognized as a safe and cost-effective procedure, which allows for accurate diagnosis, decision-making, and when indicated treatment planning. Consequently, percutaneous image-guided breast biopsies have almost entirely replaced diagnostic surgical excisions, which were associated with longer hospital stays, higher costs, and possible complications, and have become the standard of care in the medical community.

There are also different technical options for breast tissue sampling, including, for example: fine-needle sampling (FNS), core needle biopsy (CNB), and vacuum-assisted biopsy (VAB), which may be used in conjunction with various imaging modalities for guidance. These different techniques are all available to diagnose palpable and nonpalpable breast lesions, with each generally be used for a different purpose. Accordingly, the different biopsy techniques adopt needles of different sizes and lengths. The diameter of a needle's lumen (opening) is described by gauge numbers, with smaller gauge numbers indicating larger needle diameters. Commonly applied needles, for example, have a lumen diameter ranging from about 0.4 mm (27 gauge) to about 4.6 mm (7 gauge).

VAB procedures generally require needle sets having a relatively small gauge ranging from about 12 to 7 gauge. Furthermore, the standard of care for breast biopsies has shifted to utilizing smaller gauge needles. Smaller gauge needles (with larger lumen diameters) are desirable, for example, to extract larger amounts of tissue and to collect multiple samples sequentially without having to remove the needle from the patient to provide enough tissue for the pathologists to make a diagnosis, thereby reducing the risk of false-negative results or pathological underestimation. Larger needles (having smaller gauges), however, also raise issues with patient comfort, as it is generally more difficult to penetrate a patient's skin with such needles.

It may, therefore, be desirable to provide needle tips for biopsy needles and/or needle sets, including, for example, small gauge biopsy needles and needle sets, having a design that improves the patient experience and facilitates patient comfort by more easily penetrating a patient's skin to collect soft tissue samples, while also enabling large scale manufacturing of the needles and/or needle sets for commercial use. It may be further desirable to provide needle tips for biopsy needles and/or needle sets that allow for decreased insertion forces as the needle enters the soft tissue, while simultaneously not increasing the length of the needle.

### Summary

The present disclosure addresses one or more of the above-mentioned problems and/or achieves one or more of the above-mentioned desirable features. Other features and/or advantages may become apparent from the description which follows.

In accordance with various exemplary embodiments of the present disclosure, a biopsy needle for penetrating soft tissue includes an elongate body extending between a proximal end and a distal end. The elongate body has a central longitudinal axis. The biopsy needle also includes a tissue piercing tip at a distal end of the elongate body. The tissue piercing tip includes a first set of facets defined by three first faces and three first cutting edges. Each first cutting edge is formed by adjacent faces of the three first faces and each first cutting edge forms a first angle relative to the central longitudinal axis. The tissue piercing tip also includes a second set of facets positioned distal to the first set of facets. The second set of facets is defined by three second faces and three second cutting edges. Each second cutting edge is formed by adjacent faces of the three second faces and each second cutting edge forms a second angle relative to the central longitudinal axis. The second angle is smaller than the first angle. The three second cutting edges converge to form a distal end point of the biopsy needle. In an example, the biopsy needle is a nine-gage (9-G) needle. In another example, the biopsy needle is a seven-gauge (7-G) needle. In still another example, the first angle of the biopsy needle ranges between about 17 degrees and about 23 degrees relative to the central longitudinal axis, and the second angle ranges between about 9 degrees and about 15 degrees relative to the central longitudinal axis.

In another example, a diameter of the elongate body is greater than a diameter of the tissue piercing tip. In another example, the elongate body includes a sidewall defining a lumen. In yet another example, the elongate body includes a tissue receiving aperture in the sidewall, the tissue receiving aperture being positioned adjacent to the tissue piercing tip in the distal end of the elongate body. In still another example, a distance between the distal end point and the tissue receiving aperture defines a dead space, the dead space ranging from about 8 mm to about 11 mm. In one example, the first angle is about 20 degrees, the second angle is about 9 degrees, and the dead space is about 8.4 mm. In a second example, the first angle is about 20 degrees, the second angle is about 12 degrees, and the dead space is about 8.4 mm. In a third example, the first angle is about 20 degrees, the second angle is about 15 degrees, and the dead space is about 8.4 mm.

In a further example, the three first faces are all symmetrical to each other and the three first cutting edges are all symmetrical to each other. In an additional example, the three second faces are all symmetrical to each other and the three second cutting edges are all symmetrical to each other.

In another example, the tissue piercing tip comprises one or more reliefs. In still another example, the tissue piercing tip comprises three reliefs extending along the central longitudinal axis, each of the reliefs extending along a respective facet surface of the tissue piercing tip that is formed by a respective pair of first and second facets. In yet another example, each of the reliefs forms a concave surface extending along the facet surface between the cutting edges, the concave surface being defined by a radius of curvature and a lateral span. In yet another example, the radius of curvature is between about 1.9 mm to about 3.7 mm and the lateral span is between about 6.4 mm and about 8.3 mm.

In accordance with various additional exemplary embodiments of the present disclosure, a biopsy needle set for penetrating soft tissue includes an outer cannula extending between a proximal end and a distal end. The outer cannula defines a central lumen extending along a longitudinal axis. The outer cannula includes a tissue piercing tip at a distal end of the outer cannula and a tissue receiving aperture adjacent the tissue piercing tip. The tissue piercing tip includes a first set of facets defined by three first faces and three first cutting edges. Each first cutting edge is formed by adjacent faces of the three first faces and each first cutting edge forms a first angle relative to the longitudinal axis. The tissue piercing tip also includes a second set of facets positioned distal to the first set of facets. The second set of facets is defined by three second faces and three second cutting edges. Each second cutting edge is formed by adjacent faces of the three second faces and each second cutting edge forms a second angle relative to the longitudinal axis. The second angle is smaller than the first angle. The three second cutting edges converge to form a distal end point of the outer cannula. The biopsy needle set also includes an inner cannula disposed within the central lumen of the outer cannula. The inner cannula is configured to slide relative to the outer cannula to close the tissue receiving aperture. In an example, the first angle ranges between about 17 degrees and about 23 degrees relative to the longitudinal axis. In another example, the second angle ranges between about 9 degrees and about 15 degrees relative to the longitudinal axis.

In another example, the inner cannula includes an open distal end that is surrounded by an annular cutting blade. In still another example, the outer cannula includes a cutting board disposed in the central lumen in a position distal to the tissue receiving aperture, the cutting board being configured to seal the open distal end of the inner cannula when the inner cannula is in contact with the cutting board. In yet another example, the tissue piercing tip comprises one or more reliefs. In yet another example, the tissue piercing tip comprises three reliefs extending along the longitudinal axis, each of the reliefs extending along a respective facet surface of the tissue piercing tip that is formed by a respective pair of first and second facets. In yet another example, each of the reliefs forms a concave surface extending along the facet surface between the cutting edges, the concave surface being defined by a radius of curvature and a lateral span. The radius of curvature, for example, is between about 1.9 mm to about 3.7 mm and the lateral span is, for example, between about 6.4 mm and about 8.3 mm.

In an example, a biopsy device, comprises a body portion; and the biopsy needle set as described above attached to the body portion, the body portion including a drive assembly configured to drive movement of the outer cannula and the inner cannula of the biopsy needle set. In another example, the biopsy needle set is disposable. In still another example, the first angle ranges between about 17 degrees and about 23 degrees relative to the longitudinal axis, and the second angle ranges between about 9 degrees and about 15 degrees relative to the longitudinal axis.

In accordance with various further exemplary embodiments of the present disclosure, a multi-faceted soft tissue piercing element includes three distal planar cutting edges converging to form a sharp, distal point of the tissue piercing element. Each distal cutting edge forms a first angle with respect to a central longitudinal axis of the tissue piercing element. The tissue piercing element also includes three proximal planar cutting edges. Each proximal cutting edge extends proximally from a respective one of the three distal planar cutting edges. Each proximal cutting edge forms a second angle relative to the central longitudinal axis of the tissue piercing element. The second angle is larger than the first angle. In an example, the first angle ranges between about 9 degrees and about 15 degrees. In an additional example, the second angle ranges between about 17 degrees and about 23 degrees.

In another example, a maximum diameter of the multi-faceted tissue piercing element is less than or equal to a seven-gauge (7-G) needle. In still another example, the three proximal planar cutting edges extend distally from a body of a biopsy needle or trocar. In yet another example, the multi-faceted tissue piercing element further comprises three distal faces, each face positioned between two of the three distal planar cutting edges to form a distal pyramid of the tissue piercing element. In yet another example, the multi-faceted tissue piercing element further comprises three reliefs, each relief positioned between two of the three distal planar cutting edges.

Additional objects and advantages will be set forth in part in the description which follows, and in part will be obvious from the description, or may be learned by practice of the present teachings. At least some of the objects and advantages of the present disclosure may be realized and attained by means of the elements and combinations particularly pointed out in the appended claims.

It is to be understood that both the foregoing general description and the following detailed description are exemplary and explanatory only and are not restrictive of the present disclosure and claims, including equivalents. It should be understood that the present disclosure and claims, in their broadest sense, could be practiced without having one or more features of these exemplary aspects and embodiments.

### Brief Description of the Drawings

The accompanying drawings, which are incorporated in and constitute a part of this specification, illustrate some exemplary embodiments of the present disclosure and together with the description, serve to explain certain principles. These drawings depict only typical embodiments of the disclosed inventions and are not therefore to be considered limiting of its scope. In the drawings:
FIG. 1 is a partial, isometric view of an example embodiment of a needle having a distal tip portion formed in accordance with the present disclosure;
FIG. 2 is a partial, top view of the needle of FIG. 1;
FIG. 3 is a partial, side view of the needle of FIG. 1;
FIG. 4 is an enlarged view of the distal tip portion of the needle of FIG. 1;
FIG. 5 is a partial, side view of another example embodiment of a needle in accordance with the present disclosure;
FIG. 6 is a partial, side view of yet another example embodiment of a needle in accordance with the present disclosure;
FIG. 7 illustrates the relationship between an angle of a distal tip portion of a needle and the corresponding dead space of the needle;
FIG. 8 is a partial, bottom view of an alternative example embodiment of a distal tip portion of the needle of FIG. 1, in which the distal tip portion includes reliefs in accordance with the present disclosure;
FIG. 9 is a distal end view of the needle of FIG. 8;
FIG. 10 is a cross-sectional view of the needle of FIG. 8, taken through line A-A of FIG 9;
FIG. 11 is a perspective view of an embodiment of a biopsy device incorporating an example embodiment of a needle set including the needle of FIG. 1; and
FIGS. 12 and 13 are partial, perspective views of the needle set of FIG. 11.

### Detailed Description of the Illustrated Embodiments

As described above, small gauge needles, having larger lumen diameters, have become the standard of care for breast biopsies. Smaller gauge needles, for example, may extract larger amounts of tissue and collect multiple samples sequentially without having to remove the needle from the patient. Such biopsy techniques may, therefore, improve the patient experience (i.e., by reducing the number of needle sticks required during the biopsy), while also reducing the risk of false-negative results or pathological underestimation (i.e., since more tissue may be collected during the biopsy procedure). It is, however, generally more difficult to penetrate a patient's skin with such needles, as the penetration force required to insert the needle also increases with the size (i.e., diameter) of the needle. Accordingly, to improve the functionality of a biopsy device and to improve the patient experience, it is desirable that biopsy needle designs and, in particular, small gauge biopsy needle designs, minimize the forces required to insert the needle into a patient's soft tissue (i.e., are designed to have relatively small insertion forces into soft tissue). As sharper needles generally have smaller insertion forces, exemplary embodiments of the present disclosure contemplate a needle tip design that results in a sharp needle, and which may be used with both small and large gauge needles.

Embodiments of the present disclosure contemplate, for example, a distal needle tip portion having a multi-faceted tissue piercing element with three distal planar cutting edges converging to form a sharp, distal point and three proximal planar cutting edges extending proximally from a respective one of the three distal planar cutting edges. Each of the distal cutting edges may form a first angle with respect to a central longitudinal axis of the tissue piercing element and each proximal cutting edge may form a second angle relative to the central longitudinal axis of the tissue piercing element, wherein the second angle is larger than the first angle. In this manner, biopsy needles having a distal tip portion formed in accordance with the present disclose have a multi-faceted tissue piercing (penetrating) tip, such that a cross-section of the biopsy needle is minimized at the distal tip. In accordance with various example embodiments, the present disclosure contemplates that the distal tip portion described herein may be used, for example, in biopsy needles, trocars, and needle sets, each having a distal tissue piercing (penetrating) tip including a first transitional facet and a second piercing facet, wherein an angle of a transitional facet (a first angle) is greater than an angle of a piercing facet (a second angle). Further, the distal tip may include a plurality (or set) of transitional facets and a plurality (or set) of piercing facets, wherein the plurality of transitional facets are positioned immediately adjacent to the plurality of piercing facets, with the piercing facets forming the distal end point of the distal tip of the biopsy needle and being configured to pierce (penetrate) the tissue of the patient's skin. The distal needle tip described herein can be used on biopsy needles of any size (i.e., any gauge) and any configuration (e.g., can be used as the penetrating tip on any stylet, inner cannula, outer cannula, or trocar) that is used to penetrate or pierce soft tissue including, but not limited to, breast tissue.

In one example embodiment, the contemplated multi-faceted needle tips may be used in conjunction with needle sets including an elongate outer cannula or trocar that functions as a needle, and an inner cannula that is slidably disposed within the outer cannula. As described above, the elongate outer cannula has, at the distal end, a tissue piercing tip and a tissue receiving aperture, and the inner cannula has an open distal end surrounded by an annular cutting blade. In this manner, a physician may collect a tissue sample by: (1) piercing a patient's skin with the tissue piercing tip of the outer cannula, (2) positioning the receiving aperture of the outer cannula such that tissue for collection prolapses into the aperture, and (3) sliding the inner cannula within the outer cannula to close the tissue receiving aperture, thereby cutting the prolapsing tissue with the annular cutting blade of the inner cannula. Various known biopsy devices, such as those described in U.S. Patent No. 9,456,808, entitled "Biopsy Device with Automatic Biopsy Parameter Adjustment" and issued October 4, 2016; U.S. Patent No. 10,022,110, entitled "Biopsy Device" and issued on July 17, 2018; and U.S. Patent No. 8,808,200, entitled "Surgical Device and Method of Using Same" and issued on August 19, 2014, the entire contents each of which are incorporated by reference herein, may utilize such needle sets.

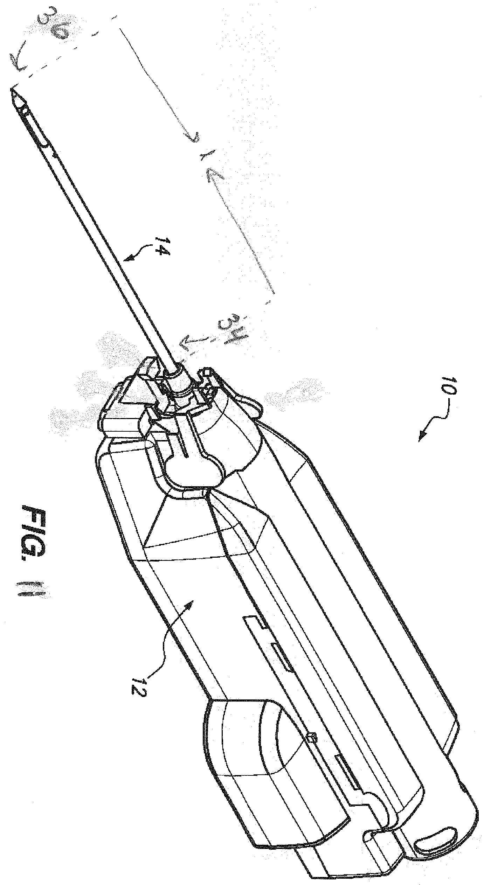

FIG. 11 illustrates a biopsy device 10 in accordance with one embodiment of the present disclosure. The biopsy device 10 includes a reusable body portion 12 and a disposable needle set 14. The body portion includes components configured to perform a tissue biopsy using the needle set 14. These components may include, for example, a drive assembly configured to drive movement of the components of the needle set 14. An exemplary drive system is described in U.S. Patent No. 10,022,110, entitled "Biopsy Device" and issued on July 17, 2018, and U.S. Patent No. 11,045,172, entitled "Biopsy Device" and dated June 29, 2021, the entire contents of each are incorporated by reference herein. The drive assembly can include one or more motors known in the art, including, for example, electrical, pneumatic, or hydraulic motors. The body portion 12 may also include a controller (e.g., a computer processor) configured to control the motors in the drive assembly and thereby control movement of the components of the needle set 14.

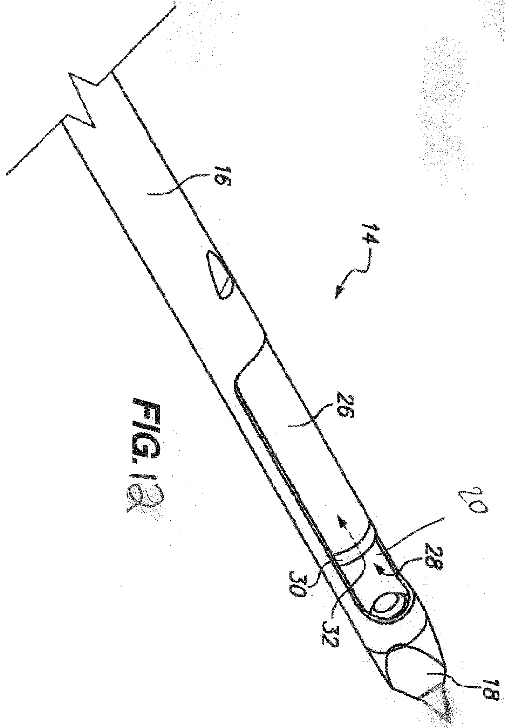

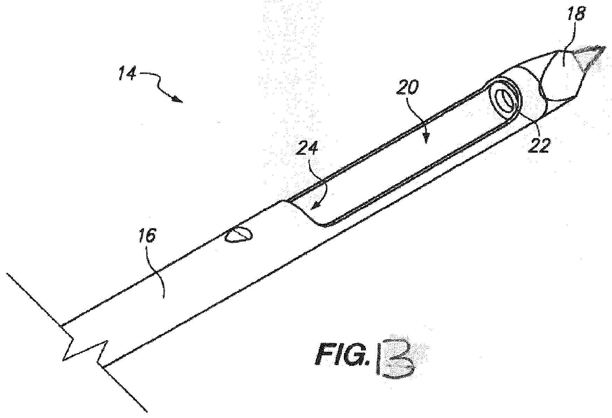

FIGS. 12 and 13 illustrate respective distal portions of the needle set 14. The needle set 14 includes an outer cannula or trocar 16 having a distal tissue piercing tip 18 formed in accordance with the present teachings and an inner cannula 26 having an open distal end 28 that is surrounded by an annular cutting blade 30. The outer cannula 16 defines an outer cannula lumen 24 and a tissue receiving aperture 20, adjacent to the distal tissue piercing tip 18, in communication with the outer cannula lumen 24. The inner cannula 26 is slidably disposed within the outer cannula lumen 24, such that when the inner cannula 26 is in its distal-most position in the outer cannula lumen 24, the inner cannula 26 closes the tissue receiving aperture 20 in the outer cannula 16. In this manner, during a biopsy procedure, the inner cannula 26 is configured to cut any tissue that extends within the receiving aperture 20 via the annular cutting blade 30 and collect the cut tissue into its open distal end 28, as the inner cannula 26 closes the tissue receiving aperture 20 of the outer cannula 16.

As shown in FIG. 13, in which the inner cannula 26 is not shown for clarity, in various exemplary embodiments, a cutting board 22 may also be disposed in the outer cannula lumen 24 in a position distal to the tissue receiving aperture 20. The cutting board 22 is configured to seal the open distal end 28 of the inner cannula 26 when the inner cannula 26 is in contact with the cutting board 22. This seal prevents fluids introduced into the outer cannula lumen 24 from being aspirated through the open distal end 28 and the inner cannula lumen 32 and bypassing the biopsy site. Instead, the fluids are delivered to the biopsy site through the outer cannula lumen 24 and the tissue receiving aperture 20.

Other aspects of the exemplary biopsy devices are described in U.S. Patent No. 9,585,639, entitled "Biopsy Device Arming Mechanism" and dated March 7, 2017; U.S. Patent No. 10,022,110; U.S. Patent No. 9,456,808; U.S. Patent No. 8,808,200; and U.S. Patent No. 9,844,363, entitled "Biopsy Device with Aspiration Valve" and dated December 19, 2017. The above-referenced patent applications are assigned to the assignee of the instant application, and the full contents thereof are hereby incorporated by reference as though fully set forth herein.

Those of ordinary skill in the art will understand that the biopsy device 10, including body portion 12 and needle set 14 illustrated in FIGS. 11-13 and described above are exemplary only, and that the multi-faceted trocars, needle tips, and needles of the present disclosure may be used in conjunction with various types and configurations of surgical instruments, biopsy devices, body portions/sleeves, stylets, needles, and needle sets, used for various types of applications, including but not limited to breast biopsies, and in various settings, without departing from the scope of the present disclosure and claims. Those of ordinary skill in the art will also understand that the disclosed needle tips (e.g., the distal tissue piercing tips) may form the distal end of an outer cannula of a needle set and/or the distal end of an inner cannula of a needle set. Furthermore, those of ordinary skill in the art will understand that the disclosed needles (e.g., cutting elements) and needle sets (e.g., needle set 14), including an outer cannula or trocar (e.g. outer cannula 16) and an inner cannula (e.g., inner cannula 26) may be made from various high strength materials, including, but not limited to surgical grade stainless steel, MP35N (nonmagnetic, nickel cobalt alloy), other cobalt-chromium alloys, NiTi alloys, ceramics, glasses, high strength polymeric materials, and/or combinations thereof.

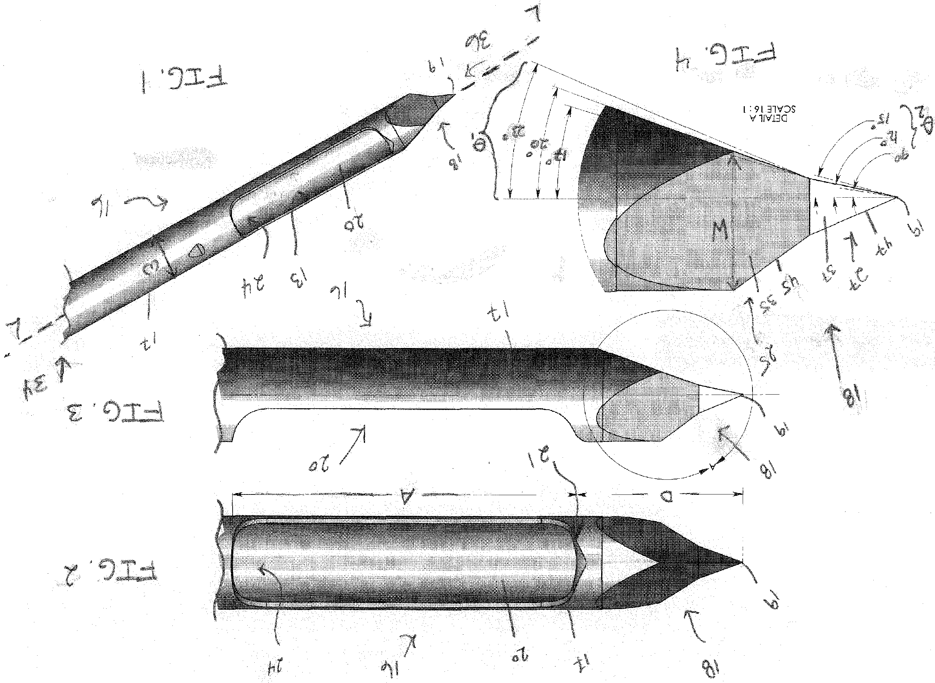

In accordance with standard practice, as will be understood by those of ordinary skill in the art, embodiments of the present disclosure contemplate that the needle tips (e.g., trocars, stylets, cannulas) of the present disclosure may be used in conjunction with various needles having various sizes and shapes (i.e., gauges and cross-sectional shapes), including, for example, needles having dimensions (e.g., widths and heights) corresponding to a 7-gauge needle through a 12-gauge needle. With reference to the embodiment of FIGS. 11-13, for example, the outer cannula 16 of the needle set 14 may have a relatively small gauge for biopsy procedures. In one example embodiment of the present disclosure, the outer cannula 16 is a 7-gauge to an 8-gauge needle, with a lumen having a width w (see FIG. 1), for example, a diameter, ranging from about 4.5 mm to about 5.6 mm. It should be noted that the range in the diameter (width) may incorporate variations between inner and outer diameters of the needle as well as variations in the cross-sectional shape of the needle. In another example embodiment, the outer cannula 16 is a 9-gauge to a 10-gauge needle, with a lumen having a width w ranging from about 3.3 mm to about 4 mm. In yet another example embodiment, the outer cannula 16 is a 11-gauge to a 12-gauge needle, with a lumen having a width w ranging from about 2.6 mm to about 3.0 mm. In various example embodiments, the cross-sectional shape of the needle may vary, for example, it may be circular or oval. Those of ordinary skill in the art will further understand, however, that the needles (trocars, stylets, cannulas) and needle sets in accordance with the present disclosure may have various gauges with various corresponding dimensions, based for example on the shape and manufacturer of the needle, to be used in conjunction with various types and configurations of surgical instruments and biopsy devices, for various types of applications, without departing from the scope of the present disclosure and claims.

FIGS. 1-4 illustrate one embodiment of a 7-gauge outer cannula or trocar 16 in accordance with the present disclosure. The outer cannula 16 includes an elongate body 17 and a tissue piercing tip 18. The elongate body 17 extends between a proximal end 34 and a distal end 36 of the outer cannula 16, such that the elongate body 17 defines a central longitudinal axis L of the cannula 16 (see FIGS. 1 and 11). The elongate body, for example, includes a sidewall 13 defining a lumen that extends along the central longitudinal axis L, with the tissue piercing tip 18 defining a distal end point 19 of the elongate body 17. Various embodiments contemplate an outer cannula having a length *l* (see FIG. 11), extending along the central longitudinal axis, ranging from about 8 mm to about 16 mm. The length *l* of a needle (i.e., outer cannula) may vary, however, depending on the needle's compatibility with a particular manufacturer and the sampling location (i.e., the location of a patient's lesion), as would be understood by those of ordinary skill in the art. As illustrated, for example, in FIG. 11 with reference to the proximal and distal ends 34 and 36 of the outer cannula or trocar 16, the terms "distal" and "proximal" refer to the orientation of a component relative to a device in which the outer cannula or trocar 16 is connected. In other words, with reference to FIG. 11, proximal signifies that a recited component is oriented closer to the body portion 12 than another component and distal signifies that a recited component is oriented further away from the body portion 12 than another component.

As best illustrated perhaps in the enlarged view of FIG. 4, to decrease the insertion forces of the outer cannula 16 (needle), the tissue piercing tip 18 of the outer cannula 16 is a multi-faceted tip, such that the distal tip 18 includes: 1) a first set of facets 25 defined by three first faces 35 and three first cutting edges 45, where adjacent faces 35 are connected to one another by cutting edges 45, and 2) a second set of facets 27 distal to the first set of facets 25, which are defined by three second faces 37 and three second cutting edges 47, where adjacent faces 37 are connected to one another by cutting edges 47. In this manner, each of the first cutting edges 45 is formed by adjacent faces 35 of the first three faces 35, and each of the second cutting edges 47 is formed by adjacent faces 37 of the second three faces 37. In various embodiments, as illustrated in FIG. 4, the three first faces 35 and the three second faces 37 are all planar surfaces, such that the first cutting edges 45 and the second cutting edges 47 are all straight (planar) cutting edges, as described further below. In various additional embodiments, the three first faces 35 and the three first cutting edges 45 are all symmetrical to each other, and the three second faces 37 and the three second cutting edges 47 are all symmetrical to each other. In various additional embodiments, the three first faces 35 and the three first cutting edges 45 are all not all symmetrical to each other, and the three second faces 37 and the three second cutting edges 47 are not all symmetrical to each other, such that the needle tip has different sized faces on either side of a cutting edges.

As further illustrated in FIG. 4, each of the first cutting edges 45 is a straight (planar) edge that forms a first angle θ₁ relative to the central longitudinal axis L (thereby creating first faces 35 each having a first tapered surface) and each of the second cutting edges 47 is a straight (planar) edge that forms a second angle θ₂ relative to the central longitudinal axis L (thereby creating second faces 37 each having a second tapered surface). The first angle θ₁ is greater than the second angle θ₂, such that the three second cutting edges 47 converge at a sharper point (i.e., at the distal end point 19 of the tissue piercing tip 18) then the first cutting edges 45 would have converged (i.e., if the tip 18 did not include the second set of facets). In this manner, a cross-section of the outer cannula 16 is minimized at the tissue piercing tip 18. In other words, the tissue piercing tip 18 is designed to include a first set of transitional facets 25 and a second set of piercing facets 27, wherein an angle θ₁ of the transitional facets 25 is greater than an angle θ₂ of the piercing facets 35, such that the piercing facets 27 form a pyramidal tip that extends distally from the transitional facets 25. As illustrated in the embodiment of FIGS. 1-4, which shows an outer cannula 16 having a first angle θ₁ of about 20 degrees and a second angle θ₂ of about 12 degrees, each first cutting edge 45 forms a first angle θ₁ ranging between about 17 degrees and 23 degrees relative to the central longitudinal axis L (the full range of the first angle θ₁ being shown in dash for illustrative purposes), and each second cutting edge 47 forms a second angle θ₂ ranging between about 9 degrees and about 15 degrees relative to the central longitudinal axis L (the full range of the second angle θ₂ being shown in dash for illustrative purposes).

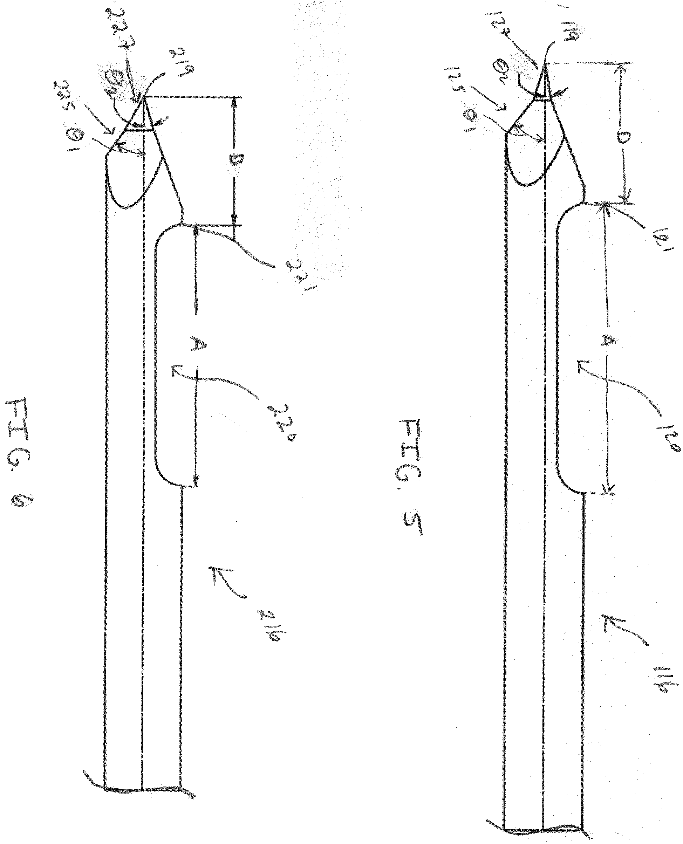

Those of ordinary skill in the art will understand that the outer cannula 16 illustrated in FIGS. 1-4 and described above is exemplary only, and that the outer cannulas (trocars or needles) in accordance with the present disclosure may have various designs and configurations, including various combinations of first sets of transitional facets and second sets of piercing facets, tapering at various combinations of first angles θ₁ and second angles θ₂ , without departing from the present disclosure and claims. FIGS. 5 and 6 contemplate and illustrate, for example, several alternative embodiments of the present disclosure, with FIG. 5 illustrating a multi-faceted outer cannula 116 having a smaller second angle θ₂ (resulting in steeper pyramidal tip and a sharper distal end point 119) in comparison to cannula 16 of FIGS. 1-4 and FIG. 6 illustrating a multi-faceted outer cannula 216 having a larger second angle θ₂ (resulting in a blunter distal end point 219) in comparison to the cannula 16 of FIGS. 1-4. The outer cannula 116 has a first set of transitional facets 125, each tapering at a first angle θ₁ of about 20 degrees, and a second set of piercing facets 127, each tapering at a second angle θ₂ of about 9 degrees. While the outer cannula 216 has a first set of transitional facets 225, each tapering at a first angle θ₁ of about 20 degrees, and a second set of piercing facets 227, each tapering at a second angle θ₂ of about 15 degrees.

As discussed above, it will also be understood by those of ordinary skill in the art that the needle tips contemplated by the present disclosure may be used in conjunction with needles having various gauges, including but not limited to the 7-gauge needles discussed above, and, for example, also contemplate using such trocars with 8 through 12-gauge needles. In one example embodiment of the present disclosure, the outer cannula is a 7-gauge to an 8-gauge needle, with piercing faucets having a width W (see FIG. 4) ranging from about 4.4 mm to about 4.8 mm and having a first angle θ₁ ranging from about 17 degrees to about 23 degrees and a second angle θ₂ of about 9 degrees to about 15 degrees. In another embodiment, the outer cannula is a 9-gauge to a 10-gauge needle, with piercing faucets having a width W ranging from about 3.1 mm to about 3.5 mm and having a first angle θ₁ ranging from about 16 degrees to about 22 degrees and a second angle θ₂ of about 8 degrees to about 14 degrees. In yet another embodiment, the outer cannula is a 11-gauge to a 12-gauge needle, with piercing faucets having a width W ranging from about 2.2 mm to about 2.6 mm and having a first angle θ₁ ranging from about 13 degrees to about 15 degrees and a second angle θ₂ of about 8 degrees to about 10 degrees.

Those of ordinary skill in the art will also understand that the tissue piercing tip of the outer cannula or trocar may be made from various materials, including, for example, surgical stainless steel (e.g., 17-4 stainless steel). And, that the transitional and piercing facets may be formed on the tissue piercing tip using various known manufacturing methods and/or techniques, including, for example, by being ground into the tissue piercing tip via, for example, a grinding wheel. In various embodiments, the faces of the facets may then be electro-polished to increase the sharpness of the cutting edges.

As discussed above, although using smaller gauge needles (needles with larger widths/diameters) offer certain benefits, it is generally more difficult to penetrate a patient's skin with such needles, as the penetration force required to insert the needle also increases with the size (i.e., diameter) of the needle. Accordingly, it is desirable that the first and second angles θ₁ and θ₂ of the tissue piercing tip be relatively small to minimize the forces required to insert the needle into a patient's tissue. Angle selection, however, also involves other considerations and factors, including, for example, a dead space D that is created by the tissue piercing tip and the fragility of the tissue piercing tip. Although not wanting to be bound by any theories, the inventors found that: (1) the range discussed above for the first angle θ₁ allows the first set of transitional facets to penetrate tissue, while not excessively increasing the dead space D of the outer cannula. In other words, the disclosed range creates a transitional portion of the distal tip of the outer cannula that results in an acceptable dead space D (as will be understood by those of ordinary skill in the art and explained further below), without requiring a user to exert excessive penetration forces to insert the outer cannula, and (2) the above discussed range for the second angle θ₂ optimizes the second set of piercing facets ability to penetrate tissue, while not making the distal end point of the outer cannula too fragile.

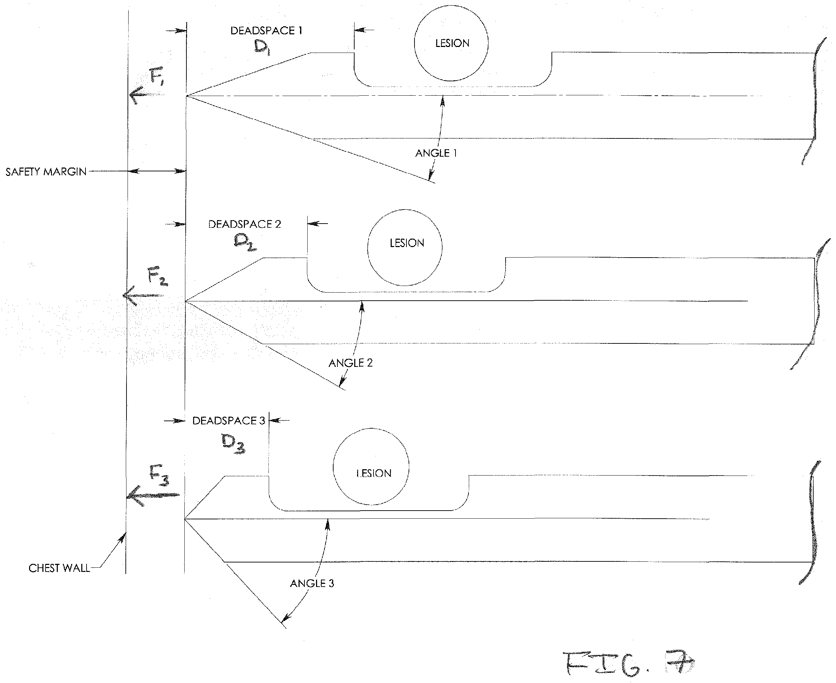

As used herein, the term "dead space" refers to a distance D between the distal end point 19, 119, 219 of the outer cannula 16, 116, 216 and a distal edge 21, 121, 221 of a tissue receiving aperture 20, 120, 220 (see FIGS. 2, 5, and 6). As illustrated in FIG. 7, when, for example, taking a biopsy of a suspected breast lesion, to allow access to tissue close to a chest wall (i.e., closer to a surface of the skin), it is advantageous to minimize the dead space D. In other words, a smaller dead space D allows access to tissue that is closer to the chest wall. It was additionally found that the dead space D is inversely related to the size of the first angle θ₁ and is therefore also inversely related to the amount of insertion force required to penetrate the skin (e.g., chest wall). Accordingly, with reference to FIG. 7: (1) a relatively small angle (angle 1) will result in a relatively small insertion force F₁ and a relatively large dead space D₁, (2) a relatively medium angle (angle 2) will result in a relatively medium insertion force F₂ and a relatively medium dead space D₂, and (3) a relatively large angle (angle 3) will result in a relatively large insertion force F₃ and a relatively large dead space D₃.

The example outer cannulas of FIGS. 1-6, which for exemplary purposes each have a first set of transitional facets 25, 125, 225 tapering at a first angle θ₁ of about 20 degrees, may each result in a dead space D of about .33 inches (8.4 mm) and have an aperture length A of about .79 inches (20 mm). Based on the present disclosure, a given application, and a sample location (e.g., legion proximity to the chest wall), one of ordinary skill in the art will be able to choose a second angle θ₂ to achieve an acceptable dead space D. For example, in embodiments of the present disclosure in which the outer cannula is a 7-gauge to an 8-gauge needle, an acceptable minimum dead space D may be about 9.5 mm to about 11 mm. And, in embodiments in which the outer cannula is a 9-gauge to a 12-gauge needle, an acceptable minimum dead space D may be about 8 mm to about 9 mm.

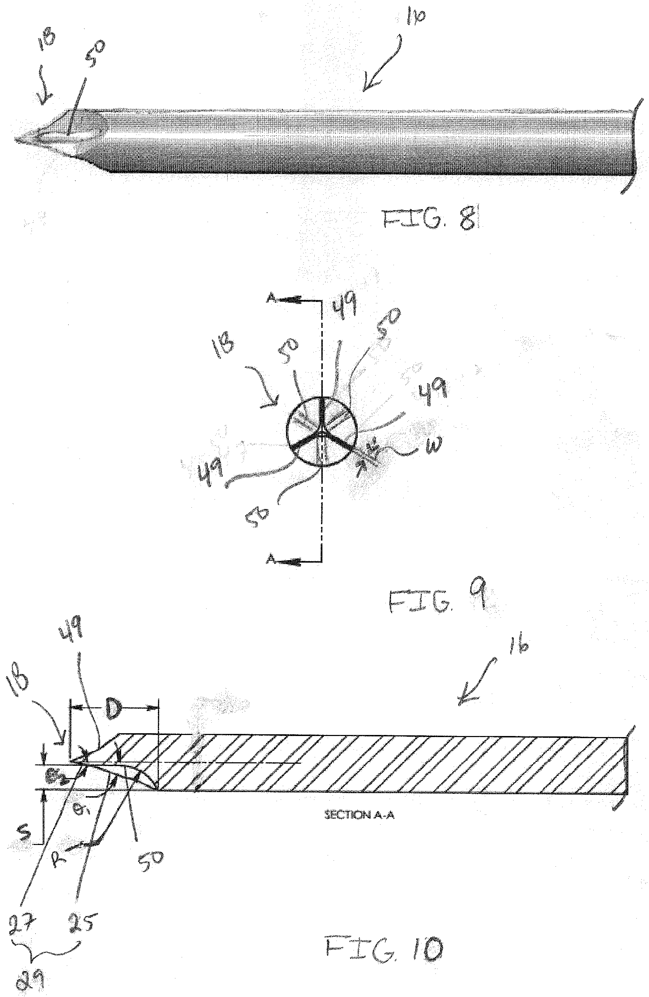

As discussed above, those of ordinary skill in the art will understand that the embodiments illustrated and described with relation to FIGS. 1-6 and 11-13 are exemplary only and that the disclosed biopsy devices, needle tips, needle sets, stylets, and inner and outer cannulas may be modified and/or include additional features without departing from the scope of the present disclosure and claims. It has also been found, for example, that the insertion force of a needle is related to the cross-sectional area of the needle tip (e.g., the tissue piercing tip 18). Accordingly, various additional embodiments of the present disclosure contemplate, for example, outer cannulas with multi-faceted tissue piercing tips (i.e., needle tips) including reliefs. As illustrated in FIGS. 8-10, in one example embodiment, the tissue piercing tip 18 of the outer cannula 16 may include one or more reliefs 50 extending along the central longitudinal axis L (three reliefs 50 are shown in the embodiment of FIGS. 8-10 (see FIG. 9)). In one embodiment, each of the reliefs 50 extends along a facet surface 29 of the tip 18 that is formed by a respective pair of transitional and piercing facets 25 and 27. As discussed above, each surface 29 is created by two different angle planes (e.g., each transitional facet tapers at a first angle θ₁ and each piercing facet tapers at a second angle θ₂), with each relief 50 creating a concave surface extending along the facet surface 29 between the planar cutting edges 45 and 47. In this manner, each relief 50 functions to remove material from the faces of the tissue piercing tip 18 to reduce the cross-sectional area of the tissue piercing tip 18, and thereby reduce the force required for insertion of the tissue piercing tip 18.

As shown best in the cross-sectional view of FIG. 10, in various embodiments, each relief 50 may have a radius of curvature R, that results in a relief 50 with a lateral span S (with relation to the central longitudinal axis L). As shown in the top view of FIG. 9, in this embodiment, removal of the three reliefs 50 forms three planar cutting edge surfaces 49 (each formed by a respective pair of cutting edges 45 and 47) each having a width w of about .01 inches (.25 mm). Taking into account the disclosed relationship of the needle tip's cross-sectional area to its insertion force, one of ordinary skill in the art will be able to choose appropriate values for the radius of curvature R, lateral span S, and cutting edge width w, for a particular application. For example, smaller values of S (e.g., as small as .03 inches), along with correspondingly larger values of R (e.g., up to 1.0 inches) and w (e.g., up to .05 inches), will result in less cross-sectional area reduction and correspondingly less insertion force reduction. In embodiments of the present disclosure in which the outer cannula is a 7-gauge to an 8-gauge needle, for example, the radius of curvature R may range from about 3.5 mm to about 3.7 mm and the lateral span S may range from about 8.1 mm to about 8.3 mm. In embodiments in which the outer cannula is a 9-gauge to an 10-gauge needle, the radius of curvature R may range from about 2.5 mm to about 2.7 mm and the lateral span S may range from about 6.4 mm to about 6.6 mm And, in embodiments in which the outer cannula is a 11-gauge to a 12-gauge needle, the radius of curvature R may range from about 1.9 mm to about 2.1 mm and the lateral span S may range from about 6.4 mm to about 6.6 mm. Those of ordinary skill in the art will understand, however, that the multi-faceted tissue piercing tips contemplated by the present disclosure may include reliefs with various configurations, positions, and/or dimensions without departing from the scope of the present disclosure and claims.

### Examples

Illustrative examples of the needle tips and sets described herein are provided below. An embodiment of the needle tip and needle set described herein may include any one or more, and any combination of, the clauses described below:
Clause 1. A biopsy needle set for penetrating soft tissue, comprising:
   an outer cannula extending between a proximal end and a distal end, the outer cannula defining a central lumen extending along a longitudinal axis, the outer cannula including a tissue piercing tip at a distal end of the outer cannula and a tissue receiving aperture adjacent the tissue piercing tip, the tissue piercing tip comprising:
      a first set of facets defined by three first faces and three first cutting edges, wherein each first cutting edge is formed by adjacent faces of the three first faces, and wherein each first cutting edge forms a first angle relative to the longitudinal axis, and
      a second set of facets positioned distal to the first set of facets, the second set of facets being defined by three second faces and three second cutting edges, wherein each second cutting edge is formed by adjacent faces of the three second faces, and wherein each second cutting edge forms a second angle relative to the longitudinal axis, the second angle being smaller than the first angle, the three second cutting edges converging to form a distal end point of the outer cannula; and
   an inner cannula disposed within the central lumen of the outer cannula, the inner cannula being configured to slide relative to the outer cannula to close the tissue receiving aperture.
Clause 2. The biopsy needle set of clause 1, wherein the first angle ranges between about 17 degrees and about 23 degrees relative to the longitudinal axis.
Clause 3. The biopsy needle set of any of clauses 1-2, wherein the second angle ranges between about 9 degrees and about 15 degrees relative to the longitudinal axis.
Clause 4. The biopsy needle set of any of clauses 1-3, wherein the inner cannula includes an open distal end that is surrounded by an annular cutting blade.
Clause 5. The biopsy needle set of clause 4, wherein the outer cannula includes a cutting board disposed in the central lumen in a position distal to the tissue receiving aperture, the cutting board being configured to seal the open distal end of the inner cannula when the inner cannula is in contact with the cutting board.
Clause 6. The biopsy needle set of any of clauses 1-5, wherein the tissue piercing tip comprises one or more reliefs.
Clause 7. The biopsy needle of clause 6, wherein the tissue piercing tip comprises three reliefs extending along the longitudinal axis, each of the reliefs extending along a respective facet surface of the tissue piercing tip that is formed by a respective pair of first and second facets.
Clause 8. The biopsy needle of clause 7, wherein each of the reliefs forms a concave surface extending along the facet surface between the cutting edges, the concave surface being defined by a radius of curvature and a lateral span.
Clause 9. The biopsy needle of clause 8, wherein the radius of curvature is between about 1.9 mm to about 3.7 mm and the lateral span is between about 6.4 mm and about 8.3 mm.
Clause 10. A biopsy device, comprising:
   a body portion; and
   the biopsy needle set of any of clauses 1-9 attached to the body portion, the body portion including a drive assembly configured to drive movement of the outer cannula and the inner cannula of the biopsy needle set.
Clause 11. The biopsy device of clause 10, wherein the biopsy needle set is disposable.
Clause 12. The biopsy device of any of clauses 10-11, wherein the first angle ranges between about 17 degrees and about 23 degrees relative to the longitudinal axis.
Clause 13. The biopsy device of clause 12, wherein the second angle ranges between about 9 degrees and about 15 degrees relative to the longitudinal axis.
Clause 14. A multi-faceted soft tissue piercing element, comprising:
   three distal planar cutting edges converging to form a sharp, distal point of the tissue piercing element, each distal cutting edge forming a first angle with respect to a central longitudinal axis of the tissue piercing element; and
   three proximal planar cutting edges, wherein each proximal cutting edge extends proximally from a respective one of the three distal planar cutting edges, each proximal cutting edge forming a second angle relative to the central longitudinal axis of the tissue piercing element, wherein the second angle is larger than the first angle.
Clause 15. The multi-faceted tissue piercing element of clause 14, wherein the first angle ranges between about 9 degrees and about 15 degrees.
Clause 16. The multi-faceted tissue piercing element of any of clauses 14-15, wherein the second angle ranges between about 17 degrees and about 23 degrees.
Clause 17. The multi-faceted tissue piercing element of any of clauses 14-16, wherein a maximum diameter of the multi-faceted tissue piercing element is less than or equal to a seven-gauge (7-G) needle.
Clause 18. The multi-faceted tissue piercing element of any of clauses 14-17, wherein the three proximal planar cutting edges extend distally from a body of a biopsy needle or trocar.
Clause 19. The multi-faceted tissue piercing element of any of clauses 14-18, further comprising three distal faces, each face positioned between two of the three distal planar cutting edges to form a distal pyramid of the tissue piercing element.
Clause 20. The multi-faceted tissue piercing element of any of clauses 14-19, further comprising three reliefs, each relief positioned between two of the three distal planar cutting edges.
Clause 21. A biopsy needle for penetrating soft tissue, comprising:
   an elongate body extending between a proximal end and a distal end, the elongate body having a central longitudinal axis; and
   a tissue piercing tip at the distal end of the elongate body, the tissue piercing tip comprising:
      a first set of facets defined by three first faces and three first cutting edges, wherein each first cutting edge is formed by adjacent faces of the three first faces, and wherein each first cutting edge forms a first angle relative to the central longitudinal axis, and
      a second set of facets positioned distal to the first set of facets, the second set of facets being defined by three second faces and three second cutting edges, wherein each second cutting edge is formed by adjacent faces of the three second faces, and wherein each second cutting edge forms a second angle relative to the central longitudinal axis, the second angle being smaller than the first angle, the three second cutting edges converging to form a distal end point of the biopsy needle.
Clause 22. The biopsy needle of clause 21, wherein the biopsy needle is a nine-gage (9-G) needle.
Clause 23. The biopsy needle of any of clauses 21-22, wherein the biopsy needle is a seven-gauge (7-G) needle.
Clause 24. The biopsy needle of clause 23, wherein the first angle ranges between about 17 degrees and about 23 degrees relative to the central longitudinal axis.
Clause 25. The biopsy needle of any of clauses 23-24, wherein the second angle ranges between about 9 degrees and about 15 degrees relative to the central longitudinal axis.
Clause 26. The biopsy needle of any of clauses 21-25 wherein a diameter of the elongate body is greater than a diameter of the tissue piercing tip.
Clause 27. The biopsy needle of any of clauses 21-26, wherein the elongate body includes a sidewall defining a lumen.
Clause 28. The biopsy needle of clause 27, wherein the elongate body includes a tissue receiving aperture in the sidewall, the tissue receiving aperture being positioned adjacent to the tissue piercing tip in the distal end of the elongate body.
Clause 29. The biopsy needle of clause 28, wherein a distance between the distal end point and the tissue receiving aperture defines a dead space, the dead space ranging from about 8 mm to about 11 mm.
Clause 30. The biopsy needle of clause 29, wherein the first angle is about 20 degrees, the second angle is about 9 degrees, and the dead space is about 8.4 mm.
Clause 31. The biopsy needle of any of any of clauses 29-30, wherein the first angle is about 20 degrees, the second angle is about 12 degrees, and the dead space is about 8.4 mm.
Clause 32. The biopsy needle of any of clauses 29-31, wherein the first angle is about 20 degrees, the second angle is about 15 degrees, and the dead space is about 8.4 mm.
Clause 33. The biopsy needle of any of clauses 21-32, wherein the three first faces are all symmetrical to each other and the three first cutting edges are all symmetrical to each other.
Clause 34. The biopsy needle of any of clauses 21-33, wherein the three second faces are all symmetrical to each other and the three second cutting edges are all symmetrical to each other.
Clause 35. The biopsy needle of any of clauses 21-34, wherein the tissue piercing tip comprises one or more reliefs.
Clause 36. The biopsy needle of clause 35, wherein the tissue piercing tip comprises three reliefs extending along the central longitudinal axis, each of the reliefs extending along a respective facet surface of the tissue piercing tip that is formed by a respective pair of first and second facets.
Clause 37. The biopsy needle of clause 36, wherein each of the reliefs forms a concave surface extending along the facet surface between the cutting edges, the concave surface being defined by a radius of curvature and a lateral span.
Clause 38. The biopsy needle of clause 37, wherein the radius of curvature is between about 1.9 mm to about 3.7 mm and the lateral span is between about 6.4 mm and about 8.3 mm.

This disclosure described some examples of the present technology with reference to the accompanying drawings, in which some of the possible examples were shown. Other aspects can, however, be embodied in many different forms and should not be construed as limited to the examples set forth herein. Rather, these examples were provided so that this disclosure was thorough and complete and fully conveyed the scope of the possible examples to those skill in the art.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

This description and the accompanying drawings that illustrate exemplary embodiments should not be taken as limiting. Various mechanical, compositional, structural, electrical, and operational changes may be made without departing from the scope of this description and the claims, including equivalents. In some instances, well-known structures and techniques have not been shown or described in detail so as not to obscure the disclosure. Furthermore, elements and their associated features that are described in detail with reference to one embodiment may, whenever practical, be included in other embodiments in which they are not specifically shown or described. For example, if an element is described in detail with reference to one embodiment and is not described with reference to a second embodiment, the element may nevertheless be included in the second embodiment.

It is noted that, as used herein, the singular forms "a," "an," and "the," and any singular use of any word, include plural referents unless expressly and unequivocally limited to one referent. As used herein, the term "include" and its grammatical variants are intended to be non-limiting, such that recitation of items in a list is not to the exclusion of other like items that can be substituted or added to the listed items.

Further, this description's terminology is not intended to limit the disclosure. For example, spatially relative terms-such as "beneath," "below," "lower," "above," "upper," "forward," "front," "behind," and the like-may be used to describe one element's or feature's relationship to another element or feature as illustrated in the orientation of the figures. These spatially relative terms are intended to encompass different positions and orientations of a device in use or operation in addition to the position and orientation shown in the figures. For example, if a device in the figures is inverted, elements described as "below" or "beneath" other elements or features would then be "above" or "over" the other elements or features. Thus, the exemplary term "below" can encompass both positions and orientations of above and below. A device may be otherwise oriented (rotated 90 degrees or at other orientations) and the spatially relative descriptors used herein interpreted accordingly.

Further modifications and alternative embodiments will be apparent to those of ordinary skill in the art in view of the disclosure herein. For example, the systems may include additional components that were omitted from the diagrams and description for clarity of operation. Accordingly, this description is to be construed as illustrative only and is for the purpose of teaching those skilled in the art the general manner of carrying out the systems and methods of the present disclosure. It is to be understood that the various embodiments shown and described herein are to be taken as exemplary. Elements and materials, and arrangements of those elements and materials, may be substituted for those illustrated and described herein, parts and processes may be reversed, and certain features of the present teachings may be utilized independently, all as would be apparent to one skilled in the art after having the benefit of the description herein. Changes may be made in the elements described herein without departing from the scope of the present disclosure.

It is to be understood that the particular examples and embodiments set forth herein are non-limiting, and modifications to structure, dimensions, materials, and methodologies may be made without departing from the scope of the present disclosure. Other embodiments in accordance with the present disclosure will be apparent to those skilled in the art from consideration of the specification and practice of the invention disclosed herein. It is intended that the specification and examples be considered as exemplary only, with being entitled to their full breadth of scope, including equivalents.

## Claims

1. A multi-faceted soft tissue piercing element, comprising:
three distal planar cutting edges converging to form a sharp, distal point of the tissue piercing element, each distal cutting edge forming a first angle with respect to a central longitudinal axis of the tissue piercing element; and
three proximal planar cutting edges, wherein each proximal cutting edge extends proximally from a respective one of the three distal planar cutting edges, each proximal cutting edge forming a second angle relative to the central longitudinal axis of the tissue piercing element, wherein the second angle is larger than the first angle.

2. The multi-faceted tissue piercing element of claim 1, wherein the first angle ranges between about 9 degrees and about 15 degrees.

3. The multi-faceted tissue piercing element of any preceding claim, wherein the second angle ranges between about 17 degrees and about 23 degrees.

4. The multi-faceted tissue piercing element of any preceding claim, wherein a maximum diameter of the multi-faceted tissue piercing element is less than or equal to a seven-gauge (7-G) needle.

5. The multi-faceted tissue piercing element of any preceding claim, wherein the three proximal planar cutting edges extend distally from a body of a biopsy needle or trocar.

6. The multi-faceted tissue piercing element of any preceding claim, further comprising three distal faces, each face positioned between two of the three distal planar cutting edges to form a distal pyramid of the tissue piercing element.

7. The multi-faceted tissue piercing element of any preceding claim, further comprising three reliefs, each relief positioned between two of the three distal planar cutting edges.
